# EUROPÄISCHE PATENTANMELDUNG

(11) **EP 2 361 653 A2**
(43) Veröffentlichungstag der Anmeldung: **31.08.2011**
(21) Anmeldenummer: 11000516.2
(22) Anmeldetag: 22.01.2011
(51) Int. Cl.: A61M 27/00

(54) **Vorrichtung zur Wundbehandlung**

(30) Priorität: 18.02.2010 DE 102010008502
(71) Anmelder: Innovations-Transfer Uphoff GmbH & Co. KG, 35039 Marburg an der Lahn (DE)
(72) Erfinder: Nink-Grebe, Brigitte, 35392 Giessen (DE)
(74) Vertreter: von Bülow, Tam

(57) **Zusammenfassung**

Die Vorrichtung zur Wundbehandlung hat zwei durch eine flüssigkeitsdichte Trennfolie (2) voneinander getrennte Wundauflagen (1, 3) und eine flüssigkeits- und luftdichte Deckfolie (5) die zumindest an ihren Rändern mit einer selbstklebenden Beschichtung (6) versehen ist. Über einen ersten Schlauch (9, 12) wird kontinuierlich Behandlungsflüssigkeit (15) aus einem Vorratsbehälter (14) zu einer der Wundauflagen (1, 3) geleitet. Ein Drainageschlauch (16) ist mit einer Pumpe (19) verbunden, die einen Unterdruck in einem Hohlraum (26) zwischen der Deckfolie (5) und einer zu behandelnden Oberfläche (25) erzeugt. Die Wundauflagen (1, 3) sind aus elastischem, offenporigem Material. Eine Steuerung (22) steuert die Pumpe (19) so, dass in dem Hohlraum (26) der Unterdruck zwischen einem oberen und einem unteren Wert variiert. Hierdurch werden die Wundauflagen (1, 3) abwechselnd komprimiert und expandiert, so dass Flüssigkeit (15) und Detrius von einer zu behandelnden Wunde (24) abgeführt werden. Die Strömungsrichtung verläuft aufgrund der Trennfolie (2) im Wesentlichen horizontal zur bzw. auf der Oberfläche der zu behandelnden Wunde (24)

## Beschreibung

Die Erfindung bezieht sich auf eine Vorrichtung zur Wundebehandlung gemäss dem Oberbegriff des Patentanspruches 1.

Eine solche Vorrichtung ist aus der US 5,636,643 A bekannt, sowie aus US 5,645,081 A1 und US 7,216,651 B1.

Aus dem Stand der Technik ist allgemein bekannt, dass der Wundverschluss im Organismus eines Säugetieres oder Menschen in der Weise geschieht, dass an die Wunde angrenzendes Epithel- und Subkutangewebe auf die Mitte der Wunde zuwandert, bis diese sich schließt. Bei großflächigen oder infizierten Wunden ist dieser Vorgang erschwert. Es bildet sich nahe der Wundoberfläche eine Stauungszone, in der eine örtliche Gewebeschwellung den Zufluss von Blut und Gewebe behindert, so dass die umgebenden Gewebeabschnitte nicht hinreichend versorgt werden. In der Folge kann eine bakterielle Infektion nicht hinreichend abgewehrt werden. Dies steht der natürlichen Wundschließung entgegen und bereitet medizinische Probleme.

Als herkömmliche medizinische Technik zum Schließen offener Wunden ist die Verwendung von Nähten oder Klammern bekannt. Dies ist zwar häufig wirksam, hat aber den erheblichen Nachteil, dass Zug auf das wundnahe Hautgewebe ausgeübt wird. Die dadurch verursachten hohen mechanischen Belastungen lassen das Gewebe reißen, was zur Wunddehiszenz (Aufplatzen) und ggf. zusätzlichen Gewebeverlusten führen kann. Auch können sich Wunden aufgrund von Infektion derart verhärten und entzünden, dass Nähen oder Klammern nicht möglich ist, und stattdessen kostenträchtige chirurgische Behandlungen notwendig sind, etwa bei grossen, tiefen und offenen Wunden, Dekubitalgeschwüren (Wundliegegeschwüre), Geschwüre aufgrund Osteomyelitis (infektiöse Entzündung des Knochenmarks) und Verbrennungen zweiten Grades, die sich zu solchen dritten Grades entwickeln können.

Daher wurden gemäss dem eingangs genannten Stand der Technik Verfahren und Vorrichtungen entwickelt, welche die Wunde durch Ausübung eines Unterdrucks drainieren. Bei diesen eingangs genannten US-Patenten bewirkt und unterstützt der applizierte Unterdruck eine Exsudation von Wundflüssigkeit, welche die Wunde feucht hält und austretendes Plasma ableitet.

Um den Unterdruck gleichmäßig auf der Wundfläche zu verteilen, werden verschiedene Wundauflagen (Schwämme, Gazen, Mischungen aus Gazen und Kunststoffen, Kompressen etc.) verwendet. Dabei kommen bereits jetzt Kunststoff-Klebefolien zur Anwendung, welche sowohl den Schwamm als auch den Unterdruck-Anschlussadapter überspannen und seitlich auf dem gesunden Gewebe fixiert werden. Dieser luftdichte Verschluss verhindert, dass atmosphärische Luft in das Unterdrucksystem eindringen kann.

Mit Hilfe eines Drainageschlauches, der den Schwamm bzw. die Kompresse mit einer Saugpumpe verbindet, wird ein Unterdruck auf die Wunde ausgeübt und Flüssigkeiten von ihr abgeleitet. Zwischen dem Drainageschlauch und der Saugpumpe befindet sich ein Sammelbehälter zur Aufnahme der Wundflüssigkeiten. Üblicherweise handelt es sich hierbei um einen Einwegbehälter.

Bei den bekannten Vorrichtungen werden zunächst Wundauflagen in Wundgröße geschnitten, Saugnapf oder Drainage angebracht und der Folienverschluss der Wunde sichergestellt. Danach wird die Absaugvorrichtung eingeschaltet. Der Unterdruck (Vakuum) wird üblicherweise zwischen 75 bis 400 mm Hg eingestellt. Heute werden für chronische Wunden und insbesondere Ulcus cruris Druckbereiche von 50 bis 75 mm Hg empfohlen, für Hauttransplantation 50 bis 125 mm Hg und 125 mm Hg für alle anderen Wunden. Somit stellt die so genannte "Vakuumversiegelung" ein geschlossenes Verbandsystem dar und ermöglicht eine feuchte Wundbehandlung.

Dabei wird die lokale Durchblutung gesteigert und ebenso eine Ödemreduktion, die Förderung von Neoangiogenese und Granulationsgewebsbildung sowie Anregung der Zellproliferation und ein Exsudatmanagement mit Reduktion von Wachstumsinhibitoren sowie Keimreduktion erreicht. Ingesamt kommt es zu einer schnelleren dreidimensionalen Wundverkleinerung und Annäherung der Wundränder.

Als Unterdruck wird hier der Druck eines Mediums bezeichnet, dessen Absolutwert unter dem Umgebungsdruck (in der Regel Atmosphärendruck)liegt. Werte des Unterdruckes bezeichnen einen Differenzwert (Umgebungsdruck minus Druck des Mediums). Sie haben daher positives Vorzeichen. Ein "größerer" oder "zunehmender" Unterdruck bedeutet daher, dass der Absolutwert des Druckes des Medium kleiner wird bzw. abnimmt und umgekehrt.

Als Schwämme kommen Polyuhrethanschäume zur Anwendung, welche grobporig und hydrophob sind und für sezernierende, infizierte, tiefe Wunden beworben werden, um eine schnelle Induktion der Granulationsgewebsbildung zu erreichen. Mit der Zielsetzung einer Wundkonditionierung und der Vermeidung des Einwachsens des Schwammes in den Wundgrund werden auch kleinporige, hydrophile Polyvinylalkoholschwämme für oberflächliche Wunden verwendet.

Zur Erzeugung des Unterdruckes werden Vakuumpumpen im Unterdruckbereich von > 50 mm Hg, ggf. auch mit wundnaher Druckmessung und kontinuierlichen als auch intermittierenden Behandlungsmodi verwendet. Die Therapie mit kontinuierlichem Unterdruck wird empfohlen, wenn das Management von erhöhten Exsudatmengen im Vordergrund steht. Ein intermittierender Unterdruck soll dagegen die Neoangiogenese und die Granulationsbildung unterstützen und wird z. B. für 5 Minuten aufrechterhalten, worauf eine Pause von 2 Minuten folgt. Als Wechselzeiten für den gesamten Verband wird für infizierte Wunden bei Einsatz von Polyuhrethanschäumen ein Zeitraum von 48 bis 72 Stunden angesetzt und für saubere Wunden bei Einsatz von Polyvinylalkoholschwämmen ein Zeitraum von 4 bis 5 Tagen.

Zur Effizienzsteigerung der Unterdrucksbehandlungsmethode wurde von M. Russ und W. Fleischmann "Die automatisierte Instillationstherapie mit VAC-Instill zur Infektsanierung" (www.a-w-a.at/pdf/jahrestagung2004/03.pdf) vorgeschlagen, wobei das angelegte Vakuum zur Instillation eines Medikamentes (z.B. Antiseptikum oder lokales Antibiotikum) für einen definierten kurzen Zeitraum unterbrochen wird. Das Medikament wird entsprechend seiner Wirkzeit im Schwamm und damit auf der Wunde belassen. Ein entsprechendes Gerät wird von der Firma KCI Medizinprodukte GmbH, Hagenauerstrasse 47, 65203 Wiesbaden unter der Bezeichnung "V.A.C. Instill Therapy Einheit" angeboten. Bei dieser "Instillationstherapie" wird der Schwamm durch ein automatisiertes System mit Lösungen, wie z.B. steriler, isotoner Kochsalzlösung, Ringerlösung, Antiseptika oder Antibiotika getränkt bzw. befüllt. Nach einer bestimmten Einwirkzeit - ohne Unterdruck - wird die installierte Lösung dann wieder durch das erneut eingeschaltete Vakuum abgesaugt, wobei diese Wechselbehandlung regelmäßig wiederholt wird.

Bekannte Risiken der derzeit eingesetzten Unterdrucksysteme sind die Bildung kontaminierten Exsudates sowie von Hautnekrosen unter der Wundauflage, welche zu schweren Infektionen führen können. Dies kann auftreten, wenn durch die zum Teil hohe Viskosität der abzusaugenden, stark eiweißhaltigen Exsudates ein vollständiger oder teilweiser Stau auftritt, weil die Wunde selbst (z. B. lokal) kein Exsudat produziert und eine angeschlossene Spülflüssigkeit diesen Wundbereich nicht überströmt. Dadurch wird die Funktion des Systems eingeschränkt und die Infektionswahrscheinlichkeit erhöht. Als Folge könne sich (Eiweiß-) Agglomerate (Fibrine, Blut, Detritus etc.) bilden, welche den Exsudatabfluss be- bzw. verhindern, so dass es zum Effekt der "Straßenbildung" kommt. Dabei werden die verfestigten Eiweiße aus dem betroffenen Bereich unterhalb der Wundauflage nicht mehr entfernt und Infektionserreger dort im Wachstum begünstigt.

Aufgabe der Erfindung ist es, die Vorrichtung der eingangs genannten Art dahingehend wesentlich zu verbessern, dass die oben genannten Nachteile vermieden werden und insbesondere eine kontinuierliche Überströmung des gesamten Wundbereiches mit der zugeführten Flüssigkeit und ein verbesserter Exsudatabfluss erreicht wird.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Die Grundidee der Erfindung liegt darin, zwei durch eine Trennfolie getrennte Wundauflagen (Schwämme, Gazen etc.) zu verwenden. Damit wird einer Behandlungsflüssigkeit eine im Wesentlichen parallel zur zu behandelnden Oberfläche verlaufende Strömungsrichtung aufgezwungen, bevor die Flüssigkeit von der ersten Wundauflage zur zweiten Wundauflage abfließen kann.

Im Folgenden wird die Erfindung anhand eines Ausführungsbeispieles im Zusammenhang mit der Zeichnung ausführlicher erläutert. Es zeigt:
- Fig. 1: eine Prinzipskizze der Vorrichtung nach der Erfindung bei einem ersten Wert des Unterdruckes und
- Fig. 2: einen Teil der Vorrichtung nach Fig. 1 bei einem zweiten Wert des Unterdruckes.

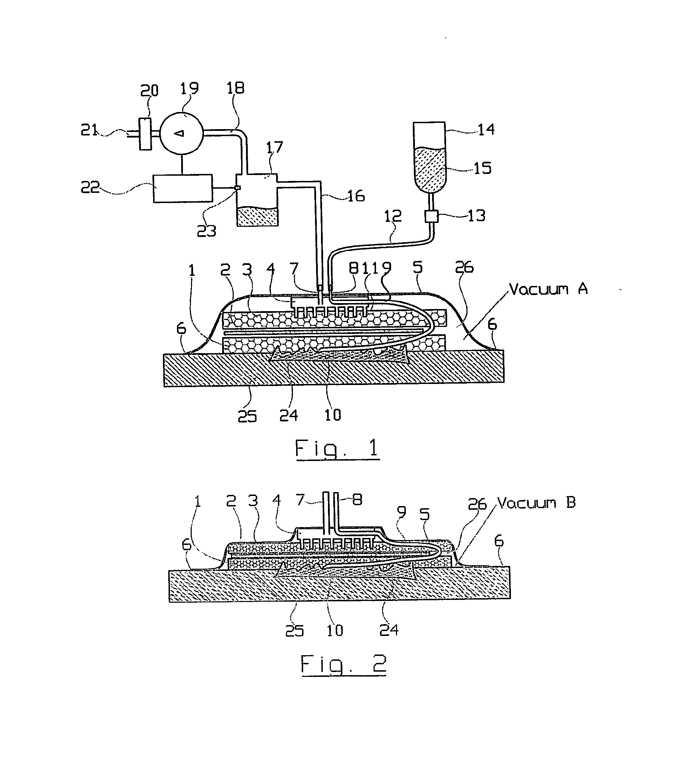

Die Vorrichtung der Fig. 1 hat eine erste Wundauflage 1, eine darüber angeordnete Trennfolie 2 und eine über der Trennfolie 2 angeordnete zweite Wundauflage 3. Beide Wundauflagen können beispielsweise aus Polyuhrethan oder Polyvinylalkohol, anderen Kunststoffen oder Gaze bestehen. Sie weisen Elastizitätsmodule zwischen 0,005 und 0,1 MPa auf. Die Wundauflagen sind offenporig um Flüssigkeiten aufnehmen und speichern zu können. Bevorzugt sind dabei grobporige Schwämme. Weiter können die Wundauflagen wahlweise hydrophil oder hydrophob sein.

Die Trennfolie 2 ist aus flexiblem und flüssigkeitsundurchlässigem Material.

Auf der der Trennfolie 2 abgewandten Oberseite der zweiten Wundauflage 3 ist ein Adapter 4 angeordnet und über dem Adapter 4 ist eine Abdeckfolie 5 angebracht, die die bisher beschriebenen Komponenten, nämlich die erste und zweite Wundauflage 1 und 3, die Trennfolie 2 und den Adapter 4 überdeckt. Die Abdeckfolie 5 ist aus flexiblem, flüssigkeits- und luftdichtem Material und zumindest an ihren Rändern mit einer selbstklebenden Beschichtung 6 versehen. Es wird als vorteilhaft angesehen, wenn die selbstklebenden Beschichtung 6 nur an ihren Rändern und im mittleren Bereich des Adapters 4 vorgesehen wird, der mit der zweiten Wundauflage 3 in Berührung kommende Bereich der Abdeckfolie 5 dagegen keine selbstklebenden Beschichtung hat.

Der Adapter 4 hat zwei Schlauchanschlüsse 7 und 8, die durch die Deckfolie 5 hindurchragen, jedoch so, dass die erwähnte Dichtigkeit der Deckfolie 5 erhalten bleibt, was beispielsweise dadurch erreicht werden kann, dass die Deckfolie 5 abdichtend mit der Oberseite des Adapters 4 verbunden, insbesondere verklebt ist. An den Adapter 4 ist ein Teilstück 9 eines ersten Schlauches angeschlossen, wobei dieses Teilstück 9 so verlegt ist, dass sein distales Ende 10 etwa in der Mitte der ersten Wundauflage 1 liegt und zwar möglichst nahe oder an dessen der Trennfolie 2 abgewandten Unterseite. Das proximale Ende dieses Teilstückes 9 steht in Strömungsverbindung mit dem Schlauchanschluss 8. Damit dieses Teilstück 9 entsprechend verlegt werden kann, sind die beiden Wundauflagen 1 und 3 je mit einem Schlitz versehen.

Der Adapter 4 hat an seiner zur zweiten Wundauflage 3 weisenden Unterseite eine Vielzahl von Öffnungen 11 oder röhrförmigen Vorsprüngen, die mit dem Schlauanschluss 7 in Strömungsverbindung stehen und damit auch mit der zweiten Wundauflage 3.

An den Schlauchanschluss 8 ist ein zweites Teilstück 12 des genannten ersten Schlauches angeschlossen, der über einen Tropfregler 13 mit einem Vorratsbehälter 14 für eine Flüssigkeit 15 verbunden ist.

An den anderen Schlauchanschluss 7 des Adapters 4 ist ein Drainageschlauch 16 angeschlossen, der zu einem Sammelbehälter 17 führt. Der Sammelbehälter 17 ist über einen weiteren Schlauch 18 mit einer Pumpe 19 verbunden, die den beschriebenen Unterdruck erzeugt. Ein Ausgang der Pumpe 19 ist über einen Filter 20 mit einem Auslass 21 zur Atmosphäre verbunden. Die Pumpe 19 wird über eine elektronische Steuerung 22 angesteuert, wobei die Steuerung 22 mit einem Drucksensor 23 verbunden ist, der den Druck bzw. Unterdruck in dem Sammelbehälter 17 misst. Selbstverständlich kann der Drucksensor 23 auch an anderer Stelle angeordnet sein, um den Unterdruck im System zu messen.

Im Folgenden wird die Arbeitsweise der Vorrichtung der Fig. 1 beschrieben. Eine zu behandelnde Wunde 24 im Gewebe 25 eines Patienten soll versorgt werden. Hierzu werden bei Bedarf zunächst die beiden Wundauflagen 1 und 3 und die Trennfolie 2 auf solche Größe und Form geschnitten, dass die Wunde 24 und deren Randbereich vollständig abgedeckt werden. Sofern die beiden Wundauflagen 1 und 3 noch keine Schlitze für das Teilstück 9 haben, werden diese ebenfalls eingeschnitten. Darauf wird das Teilstück 9 so in diese Schlitze eingelegt, dass das distale Ende 10 des Teilstückes 9 im mittleren Bereich der Wunde 24 liegt. Nach Aufsetzen des Adapters 4 auf die Oberseite der zweiten Wundauflage 3 wird die Deckfolie 5 aufgebracht und an ihren Rändern mit der selbstklebenden Beschichtung 6 an der Haut des Patienten, d.h. der Außenfläche des Gewebes 25 angeklebt. Damit entsteht zwischen der Oberfläche des Gewebes 25 und der Deckfolie 5 ein luft- und flüssigkeitsdichter Hohlraum 26. Sodann werden die Schläuche 12 und 13 an die Schlauchanschlüsse 7 und 8 angeschlossen und mit dem Vorratsbehälter 14 bzw. dem Sammelbehälter 17 verbunden.

Über den Tropfregler 13 wird dann kontinuierlich Flüssigkeit 15 über die Schlauchstücke 12 und 9 zur Wunde 24 geleitet. Über die Steuerung 22 wird die Pumpe 19 aktiviert, die in den Hohlraum 26 ein Vakuum erzeugt, das beispielsweise 120 mm Hg beträgt. Aufgrund dieses Unterdruckes bzw. Vakuums werden die beiden Wundauflagen 1 und 3 komprimiert, wie in Fig. 2 dargestellt. Dieses Vakuum wird durch die Pumpe 19 relativ rasch aufgebaut, wobei der Betrieb der Pumpe 19 angehalten wird, wenn der Drucksensor 23 feststellt, dass ein voreingestellter erster Unterdruck A erreicht ist.

Es beginnt ein kontinuierlicher Flüssigkeitseinstrom der Flüssigkeit 15 über die Teilstücke 9 und 12. Diese Flüssigkeit 15 verteilt sich über die gesamte Oberfläche der Wunde 24 und zunächst in der ersten Wundauflage 1.

Durch den kontinuierlichen Flüssigkeitsstrom steigt der Druck in dem Hohlraum 26, d.h. das Vakuum verringert sich, und die beiden Wundauflagen 1 und 3 expandieren aufgrund ihrer Eigenelastizität. Sobald der Druckanstieg einen vorbestimmten zweiten Wert B erreicht hat, der beispielsweise bei einem Vakuum von 40 mm Hg liegt, schaltet die Steuerung 22 die Pumpe 19 wieder ein und das Vakuum wird wieder verstärkt aufgebaut, bis wiederum der erste Wert A von beispielsweise 120 mm Hg erreicht ist. Dadurch werden die beiden Wundauflagen 1 und 3 wieder komprimiert. Dieser Vorgang wird zyklisch wiederholt wobei - wie erwähnt - ein kontinuierlicher Flüssigkeitseinstrom von beispielsweise 1 bis 4 Liter pro Tag aufrechterhalten wird. Die Flüssigkeitsmenge pro Zeiteinheit kann dabei entsprechend den Druckverhältnissen im Hohlraum 26 schwanken. Es kann aber auch statt eines Tropfreglers 13 eine geregelte Pumpe vorgesehen sein, die für einen konstanten Flüssigkeitseinstrom pro Zeiteinheit sorgt.

Aufgrund des beschriebenen Pumpeffektes der beiden Wundauflagen 1 und 3 und den kontinuierlichen Zufluss der Flüssigkeit 15 wird Detritus (Eiweißagglomerate, Abschilferungen, Thrombusmaterial, kleine Wundpartikel etc.) von der Wunde fortgeschwemmt und in die offenporige ersten Wundauflage 1 aufgesaugt, d.h. von der Wundoberfläche in den Innenbereich der Wundauflage abgeführt. Aufgrund der Trennfolie 2 wird jedoch verhindert, dass der genannte Detritus direkt von der ersten Wundauflage 1 in die zweite Wundauflage 3 gelangt. Vielmehr wird der Flüssigkeit 15 zusammen mit dem Detritus eine im wesentlichen parallel zur Wundoberfläche verlaufende Strömungsrichtung aufgeprägt und erst am Rand der Trennfolie 2 können diese Stoffe in die zweite Wundauflage 3 überführt werden und von dort über die Öffnungen 11, den Adapter 4, den Schlauchanschluss 7 und den Drainageschlauch 16 abgesaugt werden und in den Sammelbehälter 17 gelangen. Eventuell im Hohlraum 26 oder dem Drainageschlauch 16 vorhandene Luft wird über die Pumpe 19 und dem Filter 20 zum Auslass 21 und damit zur Atmosphäre befördert.

Die beiden Wundauflagen 1 und 3 müssen selbstverständlich gegenüber den einzusetzenden antiseptischen Wirkstoffen (wie z.B. Polyhexanid, PVP-Jod, Octenidin, Wasserstoffperoxid, Antibiotika etc.) und der Flüssigkeit 15 sowohl beständig sein als auch insbesondere keine Änderungen der Elastizität, d. h. der mechanischen Federfunktion der beiden Wundauflagen 1 und 3, verursachen.

Das Porenvolumen der Wundauflagen 1 und 3 und insbesondere der ersten Wundauflage 1 wird bei jeder Änderung des Druckniveaus ausgepresst und gleichzeitig wird die entsprechende Lösung in Richtung der Öffnungen 11 des Adapters 4 geleitet. Durch die mechanische Bewegung der ersten Wundauflage 1 auf der Wundoberfläche findet auch eine Verdünnung der Wundexsudate statt, was einer Agglomeration von beispielsweise Eiweißen entgegenwirkt.

Mechanisch wirken die beiden Wundauflagen 1 und 3 unter der Deckfolie 5 wie ein Rückstell-Federsystem, das den Unterdruck "speichert". Hierfür müssen die beiden Wundauflagen 1 und 3 definierte Druckfestigkeiten mit Elastizitätsmodulen von ca. 0,005-0,1 MPa, bevorzugt 0,02-0,05 MPa aufweisen.

Weiterhin kann die Rückstellkraft dieses Federsystems durch Einsatz verschiedener Stärken der Wundauflagen beeinflusst werden. Auch lässt sich über die Höhe der

Wundauflagen und das sich daraus ergebende Volumen, das während des Zeitraumes des Druckabfalls angesaugte Lösungsvolumen beeinflussen. Der zeitliche Zustrom der Flüssigkeit 15 muss in Abhängigkeit von der jeweiligen Wundart eingestellt werden. Die Regelung des Volumenstromes kann durch den Tropfregler 3 bestimmt werden aber auch durch Ventile, einer Pumpe oder die Verwendung von Kapillaren.

Der kontinuierliche Flüssigkeitsstrom bis unmittelbar auf die Wundoberfläche bringt in Verbindung mit der Unterdruckbehandlung wesentliche Vorteile gegenüber dem Stand der Technik. Neben der gleichmäßigen überströmenden Befeuchtung der Wunde, welche von der Exsudatbildung der Wunde unabhängig ist, wird auch eine unmittelbare Verdünnung von freigesetztem Wundexsudate unmittelbar auf der Wundoberfläche erreicht. Weiterhin erlaubt die Erfindung insbesondere die Möglichkeit der Applikation von antiseptischen und Biofilm-auflösenden Lösungen auf die unmittelbare Wundoberfläche, wodurch Infektionserreger schneller von der Wundoberfläche entfernt werden und sie anschließend in verdünnter Lösung besser mit antiseptischen Wirkstoffen erreicht werden können. Hierdurch werden die Infektionserreger besser gehemmt oder abgetötet. Unter den Verdünnungseffekten, die mit der Vorrichtung nach der Erfindung erreicht werden, haben Infektionserreger eine wesentlich geringere Chance, sich in Eiweißagglomerationen zu schützen.

Die oben beschriebene Strömungsrichtung der Flüssigkeit kann auch entgegengesetzt der bisher beschriebenen Richtung verlaufen. Im Prinzip werden lediglich die Schläuche 12 und 16 vertauscht, d.h. der Schlauch 12 wird an den Schlauchanschluß 7 und der Drainageschlauch 16 an den Schlauchanschluß 8 angeschlossen. Die Flüssigkeit 15 strömt dann über den Adapter 4 in die zweite Wundauflage 3 zu dessen Rändern, tritt dort unter "Umgehung" der Trennfolie 2 seitlich in die erste Wundauflage 1 ein und gelangt dann zur Wunde 24, wo sie über das distale Ende 10 des Teilstückes 9 zum Sammelbehälter 17 abgesaugt wird.

## Patentansprüche

1. Vorrichtung zur Wundbehandlung mit einer ersten Wundauflage (1),
einer darüber angeordneten luftdichten Deckfolie (5),
einem ersten Schlauch (9, 12), dessen proximales Ende (10) mit einem Vorratsbehälter (4) verbindbar ist, und
einem Drainageschlauch (16), der mit einer Pumpe (19) zur Erzeugung eines Unterdruckes verbindbar ist,
wobei der erste Schlauch (9, 12) und der Drainageschlauch die luftdichte Deckfolie (5) durchdringen,
**dadurch gekennzeichnet,**
- **dass** zwischen der ersten Wundauflage (1) und der Deckfolie (5) eine zweite Wundauflage (3) angeordnet ist und
- **dass** zwischen der ersten Wundauflage (1) und der zweiten Wundauflage (3) eine flüssigkeitsdichte Trennfolie (2) angeordnet ist.

2. Vorrichtung nach Anspruch 1, **dadurch gekennzeichnet, dass** die erste Wundauflage (1) und die zweite Wundauflage (3) offenporig sind.

3. Vorrichtung nach Anspruch 1 oder 2, **dadurch gekennzeichnet, dass** die erste Wundauflage (1) und die zweite Wundauflage (3) unterschiedliche Dicke aufweisen.

4. Vorrichtung nach einem der Ansprüche 1 bis 3, **dadurch gekennzeichnet, dass** die erste Wundauflage (1) und die zweite Wundauflage (3) wahlweise hydrophil oder hydrophob sind.

5. Vorrichtung nach einem der Ansprüche 1 bis 4, **dadurch gekennzeichnet, dass** die erste Wundauflage (1) und die zweite Wundauflage (3) aus Polyuhrethan, Polyvinylalkohol, anderen Kunststoffen oder Gaze bzw. Gaze-Kunststoff-Gemischen bestehen, die Elastizitätsmodule zwischen 0,005 und 0,1 MPa aufweisen.

6. Vorrichtung nach einem der Ansprüche 1 bis 5, **dadurch gekennzeichnet, dass** das distale Ende (10) des ersten Schlauches (9) im mittleren Bereich der ersten Wundauflage (1) angeordnet ist.

7. Vorrichtung nach einem der Ansprüche 1 bis 5, **dadurch gekennzeichnet, dass** ein distales Ende des Drainageschlauches (16) im mittleren Bereich der ersten Wundauflage (1) angeordnet ist und das distale Ende des ersten Schlauches (9) in der zweiten Wundauflage (3) angeordnet ist.

8. Vorrichtung nach einem der Ansprüche 1 bis 7, **dadurch gekennzeichnet, dass** an den Drainageschlauch (16) in Strömungsrichtung vor der Pumpe (19) ein Sammelbehälter (17) angeordnet ist.

9. Vorrichtung nach einem der Ansprüche 1 bis 8, **dadurch gekennzeichnet, dass** eine elektronische Steuerung (22) elektrisch mit der Pumpe (19) und mit einem Drucksensor (23) verbunden ist, wobei der Drucksensor (23) den von der Pumpe (19) erzeugten Unterdruck misst, und
dass die Steuerung (22) die Pumpe (19) bei einem ersten vorbestimmten Wert (A) des gemessenen Druckes einschaltet und bei Erreichen eines zweiten vorbestimmten Wertes (B) ausschaltet.

10. Vorrichtung nach Anspruch 9, **dadurch gekennzeichnet, dass** der erste Wert (A) des Druckes in der Größenordnung von 40 mm Hg und der zweite Wert des Druckes (B) in der Größenordnung von 140 mm Hg liegt.

11. Vorrichtung nach einem der Ansprüche 1 bis 10,
**dadurch gekennzeichnet, dass** im ersten Schlauch (12) eine Einrichtung (13) zur Regelung der vom Vorratsbehälter fließenden Flüssigkeitsmenge vorgesehen ist.

12. Vorrichtung nach Anspruch 11, **dadurch gekennzeichnet, dass** die Einrichtung (13) so ausgestaltet ist, dass ein kontinuierlicher Strom von Flüssigkeit (15) aus dem Vorratsbehälter (14) abfließt.

13. Vorrichtung nach einem der Ansprüche 1 bis 12, **dadurch gekennzeichnet, dass** zwischen der Deckfolie (5) und der zweiten Wundauflage (3) ein Adapter (4) mit zwei Schlauchanschlüssen (7, 8) angeordnet ist, dass beide Schlauchanschlüsse (7, 8) in abgedichteter Weise die Deckfolie (5) durchdringen, und dass der Adapter (4) mehrere Öffnungen (11) aufweist, die mit einem der Schlauchanschlüsse (7) in Strömungsverbindung stehen.
